# EUROPEAN PATENT SPECIFICATION

(11) **EP 1 758 500 B1**
(45) Date of publication and mention of the grant of the patent: **04.11.2009**
(21) Application number: 05748176.4
(22) Date of filing: 13.06.2005
(51) Int. Cl.: A61B 5/00

(54) **COMBINED APPARATUS FOR MEASURING THE BLOOD GLUCOSE LEVEL FROM AN OCULAR FLUID**
KOMBINIERTES GERÄT ZUM MESSEN VON BLUTZUCKER AUS AUGENFLÜSSIGKEIT
APPAREIL COMBINE DE MESURE DE LA GLYCEMIE A PARTIE D'UN FLUIDE OCULAIRE

(30) Priority: 14.06.2004 EP 04013850
(43) Date of publication of application: 07.03.2007
(73) Proprietor: EyeSense AG, 4051 Basel (CH)
(72) Inventor: MÜLLER, Achim, 63762 Grossostheim (DE); HERBRECHTSMEIER, Peter, 61462 Königstein (DE); SEIFERLING, Bernhard, 63773 Goldbach (DE); HOFMANN, Wolfgang, 60487 Frankfurt am Main (DE)
(74) Representative: Isenbruck, Günter
(86) International application number: PCT/EP2005/006316
(87) International publication number: WO 2005/120334

(56) References cited:
- WO-A-02/087429

## Description

The present invention relates to an apparatus for measuring the blood glucose level from an ocular fluid during an extended period of time. The apparatus combines a device for high precision single point blood glucose measurement from the blood with a device for non-invasive blood glucose analysis from an ocular fluid.

One important aspect in the treatment of diabetes is the tight control of blood glucose levels, which requires frequent monitoring of blood glucose levels of patients so as to manage food intake and the dosage and timing of insulin injection. Currently, millions of diabetics are forced to draw blood daily to determine their blood sugar levels. The technique that is used most often is capillary blood glucose measurement (amperometric measurements using glucose oxidase or glucose dehydrogenase) for precise blood glucose determination; and HBA1C determination for long term compliance.

Capillary blood glucose measurement is invasive and people tend to perform as few measurements as possible in order to avoid the pain of picking their finger and to reduce the costs of buying the measurements strips. The HBA1C is a long term indicator representative of an average of the glucose level over the past 90 days. Therefore, the information gained therefrom has only a limited learning/teaching impact.

To alleviate the constant discomfort and inconvenience of diabetic patients, substantial effort has been expanded in the search for a non-invasive or minimally invasive technology to accurately determine blood glucose levels.

Accordingly, various non-invasive devices to measure blood glucose levels from an ocular fluid have been described in the literature wherein an ophthalmic lens is employed comprising a glucose receptor labeled with a first fluorescent label and a glucose competitor labeled with a second fluorescent label. By monitoring the change of the fluorescence intensity at a wavelength around the peak of the fluorescence of the quenchable fluorescent label, the amount of the fluorescently labeled competitor that is displaced from the receptor by glucose is measured and is used for determining the glucose concentration in an ocular fluid. WO-A-02/087429 discloses a fluorescence photometer for measuring blood glucose level from an ocular fluid which is capable of measuring simultaneously two fluorescence intensities at two different wavelengths.

However this technology presents several disadvantages:
1) Positioning of the measurement tool with respect to the eye of the patient:
   The positioning of the measurement beam must be done with an accuracy of a few micrometers. While this is perhaps possible with a static measuring system, this is so far impossible with respect to an *in vivo* measurement assembly comprising a hand-held apparatus.
2) Accuracy of calibration:
   The measurement of the eye surface with a hand-held fluorescence photometer requires a concept ensuring that only the fluorescence of the ocular fluid or the contact lens but not the background fluorescence of the underlying tissue is measured.
3) Accuracy of the measurement in order to decide the appropriate treatment.

It is therefore an object of the instant invention to propose an apparatus that is suitable to reduce the inconveniencies involved in the use of the conventional devices for determining the blood glucose level.

This object is achieved by the combined apparatus for measuring the blood glucose level from an ocular fluid as characterized by the features of the independent claim. Advantageous embodiments of the apparatus become apparent from the features of the dependent claims.

In particular, the apparatus according to the instant invention comprises
- a hand-held fluorescence photometer for determining the glucose level of the ocular fluid of the user
- a device for the amperometric or photometric determination of the blood glucose level of the user for calibration purpose,
wherein the hand-held fluorescence photometer comprises:
(a) first irradiating means for providing a pilot beam for irradiation of the eye of the user in order to excite a pupil fluorescence, i.e. a first fluorescence, comprising a first wavelength band, said pupil fluorescence traveling along a first optical path;
(b) first detecting means arranged in the first optical path for measuring the intensity of the of the pupil fluorescence within the first wavelength band;
(c) second irradiating means for providing a measurement beam for irradiation of the eye of the user in order to excite an ocular sensor which is in contact with the ocular fluid of the user and which - upon irradiation with said measurement beam - emits a second fluorescence comprising a second wavelength band, said second fluorescence traveling along a second optical path;
(d) second detecting means arranged in the second optical path for measuring the intensity of the second fluorescence within the second wavelength band;
wherein the first and second irradiating means are arranged such that the measurement beam irradiates the eye of the user under a predetermined angle and at a predetermined distance from the pilot beam, said angle being between 0° and 90°.

In an advantageous embodiment of the apparatus according to the instant invention, the predetermined angle and distance are chosen such, that once the fluorescence photometer is arranged at a proper distance and orientation in front of the eye of the user, the measuring beam impinges on the iris while the pilot beam impinges on the pupil.

In a further advantageous embodiment of the apparatus according to the instant invention, the first detecting means comprise a first detector for detecting the pupil fluorescence or first fluorescence and at least one wavelength selective element arranged in the first optical path for preventing light having a wavelength outside the first wavelength band from impinging on the first detector.

In still a further advantageous embodiment of the apparatus according to the instant invention, the second detecting means comprise at least a second detector for detecting the second fluorescence emitted by the ocular sensor and at least one wavelength selective element for preventing light having a wavelength outside the second wavelength band from impinging on the second detector.

The second detecting means may further comprise a third detector for detecting a third fluorescence emitted by the ocular sensor and at least one wavelength selective element for preventing light having a wavelength outside the third wavelength band from impinging on the third detector.

In a further embodiment of the apparatus according to the instant invention, the hand-held fluorescence photometer and the device for the amperometric or photometric determination of the blood glucose level together form a single integral device.

The single integral device may have the shape of a disk having a diameter of between 3 and 20 cm, preferably between 5 and 15 cm, and most preferably between 7 and 10 cm. The disk may have a thickness of between 1 and 7 cm, preferably between 2 and 4 cm.

In a different embodiment of the apparatus according to the instant invention, the hand-held fluorescence photometer and the device for the amperometric or photometric determination of the blood glucose level may be separate devices, that can be connected in order to be in communication with each other.

The device for the amperometric or photometric determination of the blood glucose level may then be designed as a docking station to which the hand-held fluorescence photometer can be docked.

In an embodiment of the apparatus according to the instant invention, the hand-held fluorescence photometer and the device for the amperometric or photometric determination of the blood glucose level may together define at least a measurement unit and a calibration unit.

The apparatus may further comprise a communication and contacts unit, and the calibration unit may be designed to provide a blood glucose measurement value representative of the blood glucose level, while the communication and contacts unit may be designed to communicate the blood glucose measurement value to the measurement unit. The measurement unit may be designed to self-calibrate using the communicated blood glucose measurement value.

Alternatively, the calibration unit may be designed to provide a measurement value that is forwarded to the measurement unit, while the measurement unit may be designed to convert the communicated measurement value into a blood glucose measurement value representative of the blood glucose level. The measurement unit may be designed to self-calibrate using the blood glucose measurement value.

The calibration unit and the measurement unit may then have a common digital control element which may be provided in the measurement unit. The calibration unit may comprise only an analogue measurement circuit controlled by the common digital control element.

Further advantageous embodiments or aspects will become apparent from the following detailed description of embodiments of the apparatus according to the invention with the aid of the drawings in which:
- Fig. 1: shows the basic principle of the measurement system according to the present invention
- Fig. 2: shows the schematic arrangement of the positioning of the measurement beam and of the pilot beam with respect to a patient's eye
- Fig. 3: shows the optical path of the pilot beam in one embodiment of the present invention
- Fig. 4: shows the optical path of the measurement beam in a further embodiment of the present invention
- Fig.5: illustrates both the optical path of the measurement beam and the optical path of of the pilot beam with respect to a patient's eye in a particular embodiment of the present invention
- Fig. 6: illustrates the working principle of an amperometric blood glucose measurement device

- Fig. 7: illustrates the working principle of a photometric blood glucose measurement device
- Fig. 8: shows schematically an embodiment of the present invention, in which the hand-held fluorescence photometer and the device for the amperometric or photometric determination of the blood glucose level are separate devices, that can be connected according to the principle of a docking station
- Fig. 9: illustrates schematically the electronics that may be used in the embodiment according to Fig. 8
- Fig. 10: shows schematically a further embodiment of the present invention, in which the hand-held fluorescence photometer and the device for the amperometric or photometric determination of the blood glucose level together form a single integral device
- Fig. 11: illustrates schematically an embodiment of the electronics of the device of Fig. 10
- Fig. 12: illustrates schematically a further embodiment of the electronics of the device of Fig. 10.

### Detailed description of the invention

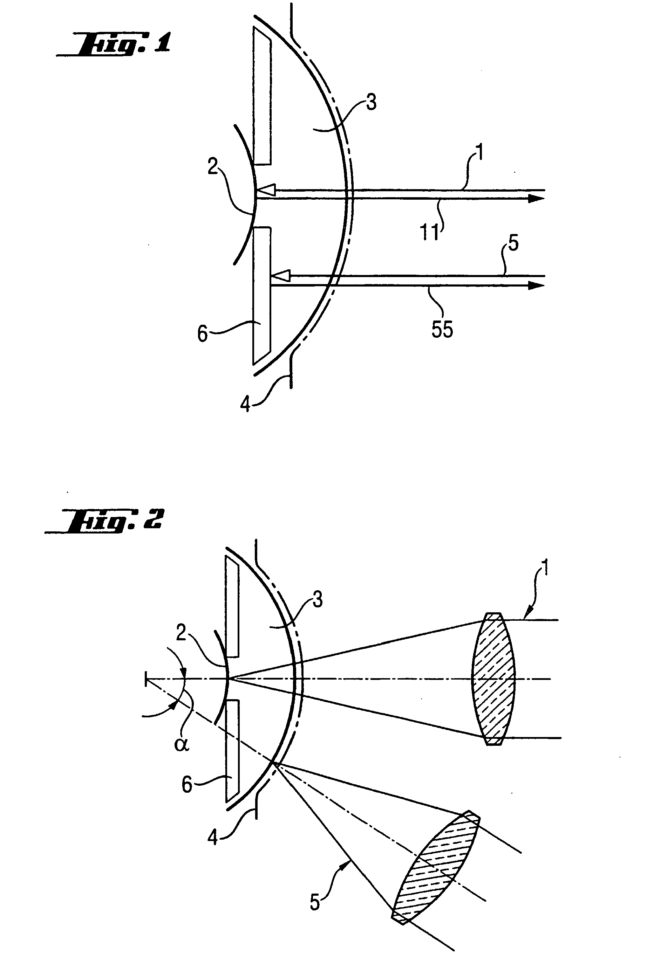

The basic working principle of an embodiment of the hand-held fluorescence photometer of the combined apparatus according to the instant invention is shown in **Fig. 1****.** First, a pilot beam 1 of a well-defined wavelength irradiates the pupil 2 of a user's eye 3 on which an ocular glucose sensor 4 is positioned. The irradiation causes a first fluorescence 11 ("pupil fluorescence") comprising a first wavelength band to be emitted through pupil 2. The first fluorescence 11 travels along a first optical path, and its intensity within the first wavelength band is measured by means of a first detecting means. The measured intensity is then correlated to the distance between the fluorescence photometer and the eye.

The geometry of the fluorescence photometer is such that pilot beam 1 and a measurement beam 5 which is used for the actual determination of the glucose level of the ocular fluid are positioned such that measurement beam 5 irradiates the eye 3 of the user under a predetermined angle α and at a predetermined distance from pilot beam 1, as shown in **Fig. 2****.** When the distance of the photometer from the eye is such that the measurement beam irradiates the iris 6 of the user, an internal electronics (not shown) sends a signal to start the actual glucose measurement. Only then, measurement beam 5 irradiates the iris 6 of the user's eye 3. Upon irradiation with measurement beam 5, ocular glucose sensor 4 emits a second fluorescence 55 comprising a second wavelength band. The second fluorescence travels along a second optical path, and its intensity is measured within the second wavelength band by means of a second detecting means. The measured intensity is then correlated to the glucose concentration in the ocular fluid of the user, which glucose concentration is then correlated to the glucose concentration of the blood of the user.

The angle α is chosen such that measurement beam 5 irradiates the surface of the eye 3 in the area of the iris 6, with the limits of iris 6 being set by the pupil 2 and the sclera. Accordingly, the optics of the photometer is chosen such that - given an optimal distance of the photometer from the eye - the aforementioned condition is fulfilled. The angle α is greater than 0° and smaller than 90°. Preferably, the angle α is between 20° and 50° and more preferably between 30° and 40°. A preferred measurement distance is between 100 mm and 1 mm, more preferably between 5 mm and 30 mm.

Pilot beam 1 also causes the emission of fluorescence from ocular sensor 4 but the intensity of this fluorescence can be neglected compared to the first fluorescence emitted through pupil 2. Analogously, measurement beam 5 causes the emission of fluorescence from iris 6, however, the intensity of this fluorescence can be neglected compared to the second fluorescence emitted from ocular glucose sensor 4.

When pilot beam 1 irradiates pupil 2 of the user's eye 3, pupil 2 becomes thus improving the measurement conditions, since pupil versus iris dimensions vary from user to user and on illumination conditions.

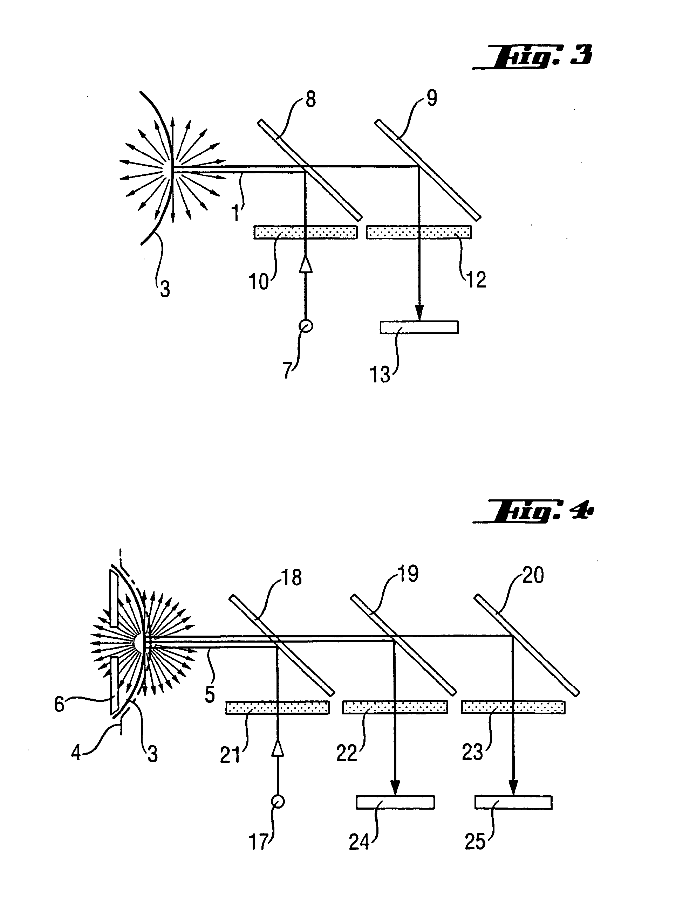

**Fig. 3** shows schematically the optical path of pilot beam 1 with respect to the eye 3 and within an embodiment of the fluorescence photometer. The fluorescence photometer comprises a first light emitting diode 7 serving as a light source, dichroic mirrors 8 and 9 each having the two functions reflecting and splitting the beam, filters 10 and 12, and a first detector 13.

The first light emitting diode 7 emits excitation light of a defined wavelength band. The excitation light passes through filter 10 so as to obtain monochromatic pilot beam 1. Dichroic mirror 8 directs pilot beam 1 towards the user's eye 3. Before hitting pupil 2 of the user's eye 3 pilot beam 1 is collimated and properly focused by means of standard lenses (not shown in Fig. 3). Such irradiation of the eye 3 causes a characteristic pupil fluorescence also referred as first fluorescence to be emitted through pupil 2, said first fluorescence travelling back towards dichroic mirror 8. Dichroic mirror 8 blocks the reflected excitation light while allowing the first fluorescence comprising higher wavelengths to pass through and to proceed further on its optical path. Dichroic mirror 9 then directs the first fluorescence towards filter 12 which allows only those wavelengths within the first wavelength band (see further above) to pass through and to reach first detector 13 in order to get measured.

**Fig. 4** shows schematically the optical path of measurement beam 5 with respect to the eye 3 and in an embodiment of the fluorescence photometer. In this embodiment, an ocular glucose sensor 4 emitting a fluorescence comprising a second fluorescence and a third fluorescence at well-defined wavelength bands is used.

The photometer comprises a second light emitting diode 17 serving as a light source, dichroic mirrors 18 and 19 each having the two functions reflecting and splitting the beam, a conventional mirror 20, filters 21, 22 and 23, as well as a second detector 24 and a third detector 25. The second light emitting diode 17 emits excitation light of a defined wavelength band which passes through filter 21 so as to obtain monochromatic measurement beam 5. Dichroic mirror 18 directs measurement beam 5 towards the user's eye 3. Before hitting the iris 6 of the user's eye 3 measurement beam 5 is collimated and properly focused by means of standard lenses (not shown). Such irradiation of the iris 6 causes the glucose ocular sensor to emit a fluorescence travelling back towards dichroic mirror 18. Dichroic mirror 18 blocks the reflected excitation light while allowing the total fluorescence comprising higher wavelength bands to pass through and to proceed further on its optical path. Dichroic mirror 19 splits the fluorescence into a second fluorescence comprising a second wavelength band and a third fluorescence comprising a third wavelength band. The second fluorescence having a lower wavelength band then the third fluorescence is directed towards filter 22 while the third fluorescence is allowed to pass through. Filter 22 allows only the those wavelengths of seccond fluorescence that are within the second wavelengths band (see further above) to reach second detector 24 in order to get measured. The third fluorescence travels towards conventional mirror 20 directing it towards filter 23 that allows only wavelengths within the third wavelength band to pass through to reach third detector 25 in order to get measured.

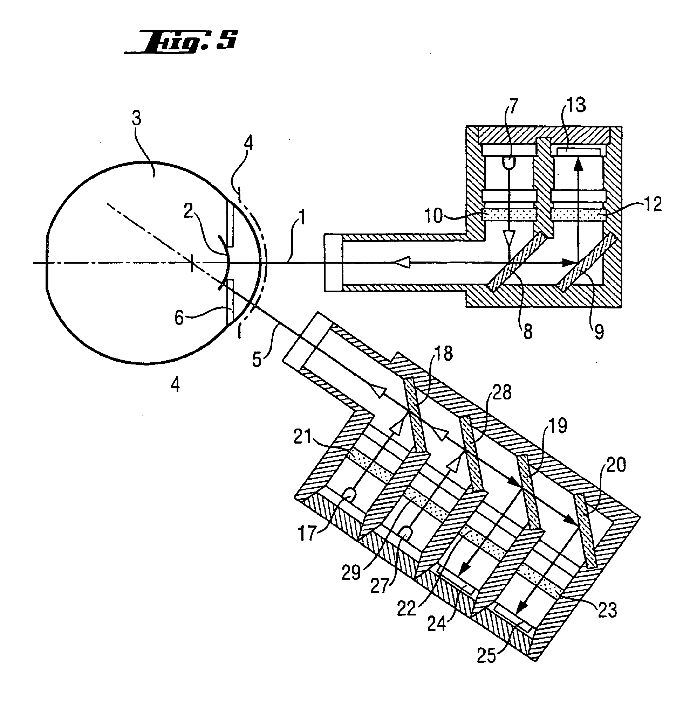

In a further embodiment more than one light source may be provided. An example of such an embodiment is illustrated in **Fig. 5****.** As can be seen, there is a third light emitting diode 27, an additional dichroic mirror 28 and an additional filter 29. The excitation light coming from second light emitting diode 17 excites especially the second fluorescence of ocular sensor 4 while the excitation light coming from third light emitting diode 27 excites especially the third fluorescence of ocular sensor 4. In the same manner as described before, dichroic mirror 28 blocks lower wavelengths and allows the higher wavelength band to pass through and to continue on its optical path.

In a further embodiment the photometer may comprises one or more additional irradiating means for providing pilot beam 1. The light sources are then preferably used sequentially during positioning of the apparatus.

Although not shown in detail, the photometer includes an electronics comprising a calculating means or a processing circuit for determining based on the measured fluorescence intensities:
- a distance between the photometer and the user's eye;
- an ocular glucose concentration in the ocular fluid of the user according to a predetermined calibration table or calibration curve;
and a means for converting the ocular glucose concentration determined by the calculating means into a blood glucose concentration by referring to a predetermined correlation between blood glucose concentrations and ocular glucose concentrations. Also, the combined apparatus may comprise a display panel for displaying the blood glucose concentration so determined.

To a person skilled in the art it will appear obvious to modify the apparatus described above in the case in which the ocular sensor emits a fluorescence with only one wavelength band or in the case in which the ocular sensor emits a fluorescence with more than two wavelength bands. For example, the number of dichroic mirrors in the optical path may be reduced or increased, accordingly. Analogously, the number of light sources may be increased, if necessary.

The light sources are preferably Surface Mounted Device light emitting diodes which are characterized by a well-defined wavelength band as well as by uniform light distribution and smaller power compared to standard light emitting diodes. Alternatively, any other kind of light emitting diodes, lasers or electroluminescence light sources may be employed.

Dichroic mirrors block lower wavelengths and allow higher wavelengths to pass through and to continue on their optical path. Their positioning with respect to the beams as well as the positioning of the filters are to be optimized for every specific arrangement.

The ocular glucose sensor 4 may, for example, emit a second fluorescence at 520 nm and a third fluorescence at 590 nm while a surface mounted light emitting diode having an excitation light of 465 nm is used. The dichroic mirrors and the filters preferably are arranged at an angle of 45° and 90° respectively relative to the pilot optical paths of the emitted fluorescence. The angle α between the irradiating pilot beam and the irradiating measurement beam may in this embodiment be 35°.

In order to cope with the required small dimensions of the photometer, the pilot beam and the measurement beam preferably may have confocal optics. To accurately position the photometer with respect to the user's eye it may be advantageous that the pilot beam has a sharp focus. To reduce the effect of eye movement during glucose measurement the measurement beam may have a more diffuse focus.

An initial calibration process correlates the blood glucose concentration with the ocular glucose concentration. The most reliable and accurate calibration method comprises determining the blood glucose concentration in the blood with a known electrochemical or optical measurement technique and correlating it with the fluorescence intensity measured in the ocular fluid of the same patient.

Both the electrochemical and optical devices known in the art for determining blood glucose employ strips. Typically, a droplet of blood is picked from the user's finger and is deposited on a strip. The strip contains an enzyme that reacts with the glucose in the blood, thus causing an enzymatic reaction. Depending on the type of chemistry involved different reaction products are released. Electrochemical devices make direct use of electrons or other charged reaction products while optical devices measure the presence of reaction products indirectly.

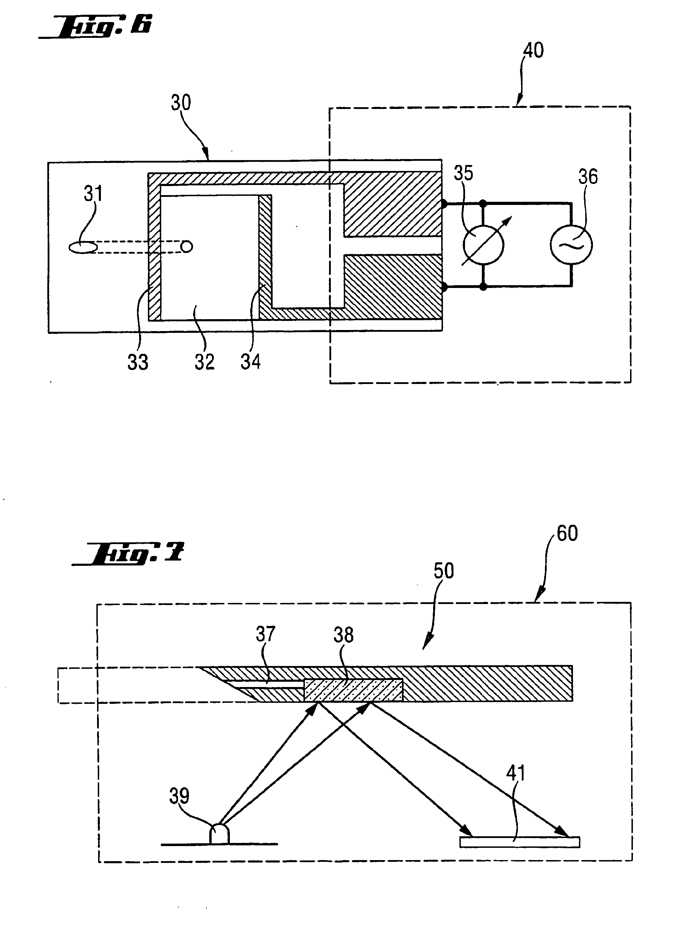

Electrochemical blood glucose measuring devices such as amperometric devices mainly use strips containing the enzyme glucose dehydrogenase. A typical amperometric glucose measuring device is schematically illustrated in **Fig. 6****.** The amperometric strip 30 comprises a capillary 31 through which the blood reaches a chemically reactive area 32 of the strip 30 where the reaction between glucose and the enzyme takes place. The strip 30 further includes two electrodes 33, 34 connected to said chemically reactive area 32. Upon introducing strip 32 into an amperometric glucosimeter 40 the charged products released during the reaction between glucose and the enzyme are separated by an electrical field that can be generated between electrodes 33 and 34 on the strip 30. By properly connecting an amperometer 35 to electrodes 33 and 34 a direct current can be measured. This direct current is proportional to the amount of glucose present on the strip. In order to accurately determine the glucose concentration, it is necessary to also determine the amount of blood in which the measured amount of glucose is contained. This can be achieved in a well-known manner by connecting the electrodes 33 and 34 to an alternating current source 36.

Optical blood glucose measuring devices such as photometric devices mainly employ strips containing the enzyme glucose oxidase. The charged products released during the reaction between glucose and the enzyme reduce a colorant which - as a consequence - changes its color. In this case the strips do not include any electrical circuit. A typical photometric blood glucose measuring device is schematically illustrated in **Fig. 7****.** The photometric strip 50 comprises a capillary 37 through which the blood reaches a chemically reactive area 38 of the strip where the reaction between glucose and the enzyme takes place. When the photometric strip 50 is introduced into a photometric glucosimeter 60 a light source 39, e.g. a light emitting diode, illuminates the chemically reactive area 38 of the strip 50 and the intensity of the reflected light having the changed color is converted to a current that is proportional to the glucose concentration in the blood by means of a photodiode 41.

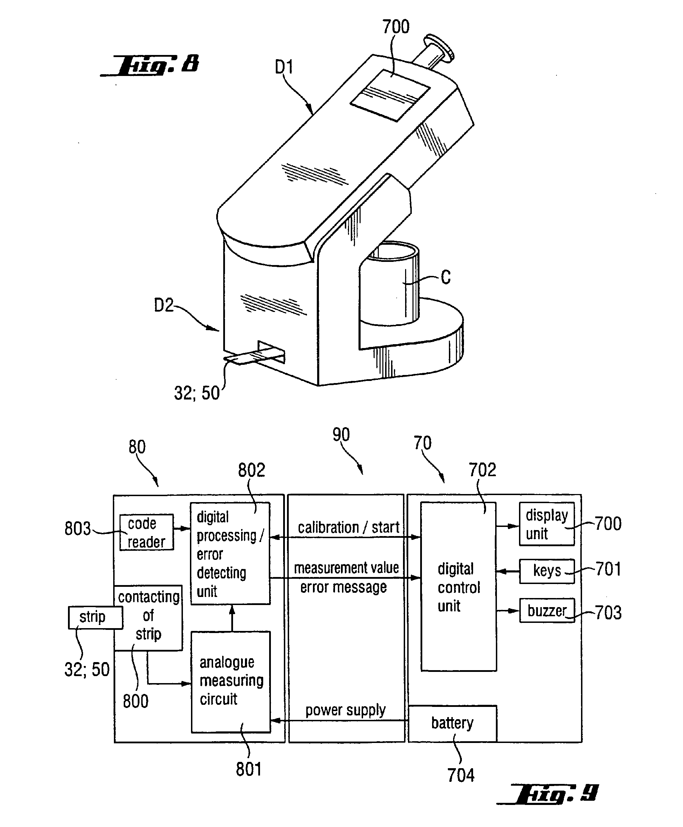

**Fig. 8** shows schematically an embodiment of the combined apparatus according to the invention. In this embodiment, the hand-held fluorescence photometer D1 for determining the glucose level of the ocular fluid and the device D2 for the amperometric or photometric determination of the blood glucose level are separate devices that can be connected to be in communication with each other. A container C for storing an inventory of measuring strips may also be provided. In general, the connection between the photometer D1 and device D2 can be achieved using any suitable interface, however, in the specific embodiment shown in Fig. 8 the device D2 for the amperometric or photometric determination of the blood glucose level serves as a docking station to which the hand-held fluorescence photometer D1 can be docked, thus establishing the connection. This kind of docking is somehow similar to the docking of a so-called "Personal Digital Assistant" (PDA) to a respective docking station. A calibration of the hand-held fluorescence photometer can be performed by measuring the glucose concentration present in the ocular fluid with the aid of hand-held fluorescence photometer D1, by introducing a strip 32 into device D2 for determining the blood glucose concentration, and by correlating the glucose concentration of the ocular fluid with the actual blood glucose concentration, thus calibrating hand-held fluorescence photometer D1. After calibration the blood glucose concentrations can be determined by solely using hand-held fluorescence photometer D1. The so determined glucose concentration is displayed on display unit 700. Calibration can be done once a month, more preferably once a week and even more preferably once a day.

On an electronic level, hand-held fluorescence photometer D1 and the device D2 for determining the blood glucose concentration together define at least a measurement unit 70 and a calibration unit 80, in the embodiment shown they also define an additional communication an contacts unit 90. This is schematically illustrated in **Fig. 9****.** Calibration unit 80 provides a blood glucose measurement value representative of the blood glucose level. Communication and contacts unit 90 communicates the blood glucose measurement value to measurement unit 70 which uses the communicated blood glucose measurement value to self-calibrate as already outlined above. A battery 704 may be contained in measurement unit 70 which also supplies calibration unit 80 as long as measurement unit 70 is docked to calibration unit 80.

Calibration unit 80 may comprise means 800 for contacting the measuring strip, an analogue measuring circuit 801, a digital processing and error detecting unit 802 and a code reader 803. Measuring unit 70 may comprise a digital control unit 702, a display unit 700, keys 701, a buzzer 703 and a battery 704.

The code reader 803 may read information provided on the strip, e.g. the type of strip, the manufacturer etc., which may assist the digital processing and error detecting unit 802 in determining the blood glucose concentration from the measurement signal provided by the analogue measuring circuit 801. The measurement value forwarded from digital processing and error dectecting unit 802 of calibration unit 80 to digital control unit 702 of measurement unit 70 is therefore directly representative of the blood glucose level and need not be converted into such value by control unit 702 of measurement unit 70. In case no measurement value can be determined, an error message is forwarded to measurement unit 70, and buzzer 703 may be activated. Accordingly, digital processing and error detecting unit 802 is a an element that is able to perform a multitude of different operations. Also, calibration and start messages may be interchanged between calibration unit 80 and measurement unit 70. The keys 701 may be used for performing various actions, e.g. for scrolling through a menu to select a specific function. Display 700 may serve to display the menu options as well as the respective determined glucose concentrations.

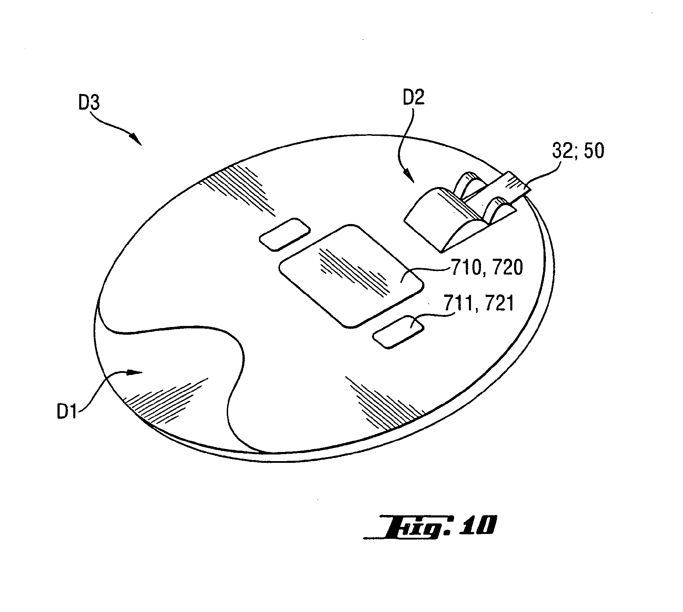

In a further embodiment of the combined apparatus according to the instant invention, illustrated in **Fig. 10****,** the hand-held fluorescence photometer D1 for determining the glucose level of the ocular fluid and the device D2 for determining the blood glucose level together form a single integral device D3. In the interior of device D3, different electronic concepts may be realized, which will be explained further below. The outer shape of device D3 corresponds more or less to a disk that may be easily carried in the pocket. In general, the working principle is similar to that of the embodiment described with the aid of Fig. 8. The glucose concentration of the ocular fluid is determined using fluorescence photometer D1, the actual blood glucose concentration is determined with the aid of a measuring strip 32 or 50, respectively. Then, the glucose concentration of the ocular fluid is correlated with the actual blood glucose concentration, thus calibrating hand-held fluorescence photometer D1. After calibration the blood glucose concentrations can be determined by solely using hand-held fluorescence photometer D1. The so determined glucose concentration is displayed on a display unit. The keys 711,721 may be used for performing various actions, e.g. for scrolling through a menu to select a specific function.

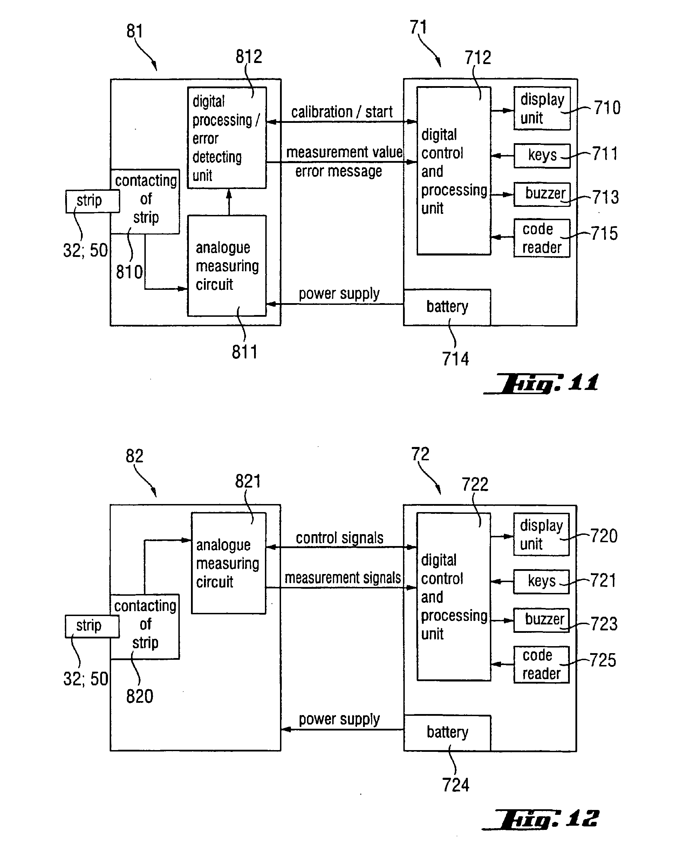

On an electronic level, hand-held fluorescence photometer D1 and the device D2 for determining the blood glucose concentration together define at least a measurement unit 71 and a calibration unit 81. No more communication and contacts unit is present, since fluorescence photometer D1 and device D2 for determining the blood glucose concentration from a single integral device D3. This is schematically illlustrated in **Fig. 11** in accordance with one concept for the electronics. Since the electronics of both devices D1,D2 can be integrated to a higher degree because of the single device D3 concept, calibration unit 81 does no longer provide a blood glucose measurement value but communicates the pure measurement value that has only been digitalized but has not been processed to directly represent the blood glucose level to measurement unit 71. The conversion from the pure measurement value to the blood glucose measurement value is done within measurement unit 71. Subsequently, measurement unit 71 uses the blood glucose measurement value so determined to self-calibrate as already outlined above. A battery may be contained in measurement unit 71 which also supplies calibration unit 81.

Calibration unit 81 may comprise means 810 for contacting the measuring strip, an analogue measuring circuit 811, and a digital processing and error detecting unit 812. Measuring unit 71 may comprise a digital control unit 712, a display unit 710, keys 711, a buzzer 713, a battery 714 and a code reader 715.

The code reader 715 may read information provided on the strip, e.g. the type of strip, the manufacturer etc., which may assist the digital processing and error detecting unit 712 in determining the blood glucose concentration from the pure measurement signal provided by the analogue measuring circuit 811. As already mentioned above, the measurement value forwarded from digital processing and error dectecting unit 812 of calibration unit 81 to digital control unit 712 of measurement unit 71 is no longer directly representative of the blood glucose level and is converted into a value directly representative of the blood glucose concentration by control unit 712 of measurement unit 71. Accordingly, the structure of digital processing and error detecting unit 812 is simpler than that of the embodiment shown in Fig. 9, thus reducing the expense for the electronics belonging to calibration unit 81. In case no measurement value can be determined, an error message is forwarded to measurement unit 71, and buzzer 713 may be activated. Also, calibration and start messages may be interchanged between calibration unit 81 and measurement unit 71.

According to a further concept of the electronics shown in **Fig. 12****,** hand-held fluorescence photometer D1 and the device D2 for determining the blood glucose concentration again define at least a measurement unit 72 and a calibration unit 82. No more communication and contacts unit is present, since fluorescence photometer D1 and device D2 for determining the blood glucose concentration form a single integral device D3. Since the electronics of both devices D1, D2 can be integrated to a higher degree because of the single device D3 concept, calibration unit 82 again does no longer provide a blood glucose measurement value but communicates a pure analogue measurement value that has not been processed to directly represent the blood glucose level to measurement unit 72. The conversion from the pure analogue measurement value into the blood glucose measurement value is done by digital control and processing unit 722 within measurement unit 72. Subsequently, measurement unit 72 uses the blood glucose measurement value so determined to self-calibrate as already outlined above. Also, the error detecting function is now performed by digital control and processing unit 722 of measurement unit 72, thus making a digital processing and error detecting unit within calibration unit 82 impossible. A battery 724 may also be contained in measurement unit 72 which also supplies calibration unit 82.

Calibration unit 82 may therefore comprise in essence only means 820 for contacting the measuring strip and an analogue measuring circuit 821. Measuring unit 72 may comprise a digital control unit 722, a display unit 720, keys 721, a buzzer 723, a battery 724 and a code reader 725.

The code reader 725 may read information provided on the strip, e.g. the type of strip, the manufacturer etc., which may assist the digital processing and error detecting unit 722 in determining the blood glucose concentration from the pure measurement signal provided by the analogue measuring circuit 821. As already mentioned above, the measurement value forwarded from analogue measuring circuit 821 to processing and error detecting unit 722 of measurement unit 82 to digital control unit 722 of measurement unit 72 is a pure analogue measurement signal that must first be converted into a digital value by digital control unit 722 of measurement unit 72, and which must then be converted also by digital control unit 722 into a digital value directly representative of the blood glucose concentration. Accordingly, a digital processing and error detecting unit can be completely omitted within calibration unit 82, thus even more reducing the expense for the electronics belonging to calibration unit 82. Also, in case no measurement value can be determined an error message must no longer be forwarded to measurement unit 72 but rather is generated within digital processing unit 722 of measuring unit 72. Buzzer 713 may then be activated. Also, calibration and start messages as well as other control signals have to be interchanged between calibration unit 82 and measurement unit 72.

In addition to this calibration, a standardization may be done measuring the fluorescence intensity of a reference dye, which may have been embedded in the ocular glucose sensor,
wherein such a dye is non-active with respect to the glucose. Whenever the ocular sensor comprises more than one fluorescent label, one could serve as an internal standard in the determination of the glucose concentration in an ocular fluid. An additional calibration may be done by measuring one fluorescent label while exciting another one. This would compensate for the variation (if any) in intensity of the pilot beam when the distance from the eye is slightly varied (order of micrometers).

As has been explained with reference to the described embodiments, the combined apparatus of the present invention may take several configurations. Preferably, an integral cover is provided to protect the optical elements. In order to exactly position the apparatus in front of the eye, the pilot beam is irradiated into the pupil of the patient's eye and the pupil fluorescence is measured. Once the photometer is exactly positioned the measurement beam irradiates the patient's eye, preferably in the region of the iris, in order to excite the ocular glucose sensor. The detected intensity of the fluorescence intensity emitted by the ocular sensor is then correlated to the glucose ocular and/or blood concentration.

The display unit preferably uses liquid crystals and/or light emitting diodes, which simplify readout of the glucose concentration value and of some instrument diagnostic functions such as, for example, battery status etc..

Further, the measured blood glucose concentration value may be transmitted to another piece of equipment via wire or cable, or wirelessly, such as via radio frequency or infrared transmission. Also, it is conceivable, that a telemetry signal can be transmitted to an infusion pump, which can provide the proper amount of insulin in order to maintain suitable levels of glucose in the body. The telemetry signal may be analog or digital.

Infusion pumps are well known in the art for delivering a selected medication to a patient including humans and other animals in accordance with an administration schedule which can be pre-selected or, in some instances, preprogrammed. Suitable pumps for use together with a combined apparatus of the instant invention can be worn externally or can be directly implanted into the body of a mammal, including a human, to deliver insulin to the mammal in controlled doses over an extended period of time. Such pumps are well known and are described, for example, in U.S. Patents 5,957,890; 4,923,375; 4,573,994; and 3,731,681.

In a preferred embodiment the calibration of the combined apparatus is done once a month, preferably once a week more preferably once a day.

A suitable ocular sensor may for example be an ophthalmic lens comprising a glucose receptor labeled with a first fluorescent label as well as a glucose competitor labeled with a second fluorescent label. The two fluorescent labels are selected such that while the competitor is bound to the receptor, the fluorescence of one of two fluorescent labels is quenched via a fluorescence resonance energy transfer by the other fluorescent label. By monitoring the change of the intensity at a wavelength around the peak of the fluorescence of the quenchable fluorescent label, the amount of the fluorescently labeled competitor that is displaced from the receptor by the glucose is measured and provides a means of determining the glucose concentration in an ocular fluid.

Fluorescent labels, such as fluorescein, indocyanine green, malachite green, and rhodamine, which are quenched when the competitor moiety is bound but are unquenched when the competitor moiety is not bound, are preferred for use as quenchable fluorescent label in the ocular glucose sensor. A particularly preferred combination of fluorescent labels is the combination of fluorescein (donor) and rhodamine (acceptor).

The sensitivity of the ocular glucose sensor can be controlled by varying the concentration of the quenchable fluorescent label. Increasing the concentration of the quenchable fluorescent label in the ocular glucose sensor increases the range of fluorescence intensity and thereby increases the sensitivity of resulting measurements.

The glucose receptor moiety comprises one or more binding sites for glucose. Each binding site may, however, also bind a moiety that competes with glucose for binding, and is therefore referred to herein as a "glucose/competitor moiety binding site". Binding of both the competitor moiety and glucose to the glucose/competitor moiety binding site is reversible. The receptor moiety can be, for example, antibodies, boronic acid, a genetically engineered bacterial fluoriprotein, or preferably concavalin A (Mansouri & Schultz, Bio/Tech 2:385 (1984)).

It is well known to a person skilled in the art to select a competitor moiety which will compete with glucose for binding to a glucose/competitor moiety binding site. For example, suitable competitors to glucose for binding to concavalin A are a polymeric carbohydrate, in particular dextran, or a glycoconjugate as described in US 5,342,789. A particular preferred receptor competitor system is a system of a labeled concavalin A and a labeled dextran, especially rhodamine-concavalin A and fluorescein dextran.

In alternative a suitable ocular glucose sensor may be an ophthalmic lens comprising a protein sensing molecule capable of binding glucose and having the property to emit - upon irradiation - a fluorescence that changes in intensity or decay time in a concentration-dependent manner when said molecule is bound to the glucose. If the glucose is glucose, preferably the protein is an *E. Coli* glucose binding protein GGBP or functionally equivalent fragments thereof. Proteins other then GGBP may be used, for example, hexokinase, glucokinase, or mutants of hexokinase or mutants of GGBP. For example, it is especially useful to modify the GGBP molecule to include cysteine residues as described in US 6,197,534. In addition, the sensing molecule may be labeled with one or more detectable labels like solvent sensitive probes such as dansyl probes, anilinonapthalene probes, deproxyl probes and similar probes which are sensitive to the polarity of the local environment . Other useful probes include donor-acceptor pairs such as fluorescein to rhodamine, coumarin to fluorescein or rhodamine. Still another class of useful label pairs include fluorophore-quencher pairs such as acrylamide groups, iodine and bromate etc in which the second group is a quencher that decreases the fluorescence intensity of the fluorescent group.

A suitable ocular glucose sensor may in addition comprise a reference dye, e.g. for standardization or calibration purposes, which upon irradiation emits a characteristic fluorescence, wherein such a dye is non-active with respect to the glucose.

An ophthalmic lens may, for example, be a removable lens such as a contact lens, or a permanently implanted lens, such as an intraocular lens, a subconjunctival lens, or an intracorneal lens. Permanently implanted lenses are particularly well-suited for use in individuals who have compromised ocular function (e.g. cataracts) and also diabetic disease.

Ophthalmic lenses can be corrective lenses or can be constructed so that they do not affect visual acuity. Contact lenses optionally can comprise a tint and are preferably disposable, which reduces the risk of infection for the user. As used herein, the term "ophthalmic lens" may also refer to a shunt or implant that may rest in the subconjunctival part of the eye.

Ophthalmic lenses according to embodiments of the invention can be worn continuously or over extended periods of time to provide repeated measurements or can be worn for a single measurement only. Both qualitative and quantitative measurements can be performed.

## Claims

1. Combined apparatus (D1, D2; D3) for measuring the blood glucose level from an ocular fluid of a user, comprising
- a hand-held fluorescence photometer (D1) for determining the glucose level of the ocular fluid of the user
- a device (D2) for the amperometric or photometric determination of the blood glucose level of the user for calibration purpose,
wherein the hand-held fluorescence (D1) photometer comprises:
(a) first irradiating means (7,8,10) for providing a pilot beam (1) for irradiation of the eye (3) of the user in order to excite a pupil fluorescence, i.e. a first fluorescence, comprising a first wavelength band, said pupil fluorescence traveling along a first optical path;
(b) first detecting means (8,9,12,13) arranged in the first optical path for measuring the intensity of the pupil fluorescence within the first wavelength band;
(c) second irradiating means (17,18,21) for providing a measurement beam (5) for irradiation of the eye (3) of the user in order to excite an ocular sensor (4) which is in contact with the ocular fluid of the user and which - upon irradiation with said measurement beam - emits a second fluorescence comprising a second wavelength band, said second fluorescence traveling along a second optical path;
(d) second detecting means (18,19,20,22,23,24,25) arranged in the second optical path for measuring the intensity of the second fluorescence within the second wavelength band;
wherein the first and second irradiating means are arranged such that the measurement beam (5) irradiates the eye of the user under a predetermined angle (α) and at a predetermined distance from the pilot beam (1), said angle (α) being between 0° and 90°.

2. Apparatus according to claim 1, wherein the predetermined angle (α) and distance are chosen such, that once the fluorescence photometer (D1) is arranged at a proper distance and orientation in front of the eye of the user, the measuring beam (5) impinges on the iris (6) while the pilot beam impinges on the pupil (2).

3. Apparatus according to any one of the preceding claims, wherein the first detecting means comprise a first detector (13) for detecting the pupil fluorescence or first fluorescence and at least one wavelength selective element (8,9,12) arranged in the first optical path for preventing light having a wavelength outside the first wavelength band from impinging on the first detector (13).

4. Apparatus according to any one of the preceding claims, wherein the second detecting means comprise at least a second detector (24) for detecting the second fluorescence emitted by the ocular sensor (4) and at least one wavelength selective element (18,19,22) for preventing light having a wavelength outside the second wavelength band from impinging on the second detector (24).

5. Apparatus according to claim 4, wherein the second detecting means further comprise a third detector (25) for detecting a third fluorescence emitted by the ocular sensor (4) and at least one wavelength selective element (18,19,20,23) for preventing light having a wavelength outside the third wavelength band from impinging on the third detector (25).

6. Apparatus according to any one of the preceding claims, wherein the hand-held fluorescence photometer (D1) and the device (D2) for the amperometric or photometric determination of the blood glucose level together form a single integral device (D3).

7. Apparatus according to claim 6, wherein the single integral device (D3) has the shape of a disk having a diameter of between 3 and 20 cm, preferably between 5 and 15 cm, and most preferably between 7 and 10 cm, the disk having a thickness of between 1 and 7 cm, preferably between 2 and 4 cm.

8. Apparatus according to any one of claims 1 to 5, wherein the hand-held fluorescence photometer (D1) and the device (D2) for the amperometric or photometric determination of the blood glucose level are separate devices, that can be connected in order to be in communication with each other.

9. Apparatus according to claim 8, wherein the device (D2) for the amperometric or photometric determination of the blood glucose level is designed as a docking station to which the hand-held fluorescence photometer (D1) can be docked.

10. Apparatus according to any one of the preceding claims, wherein the hand-held fluorescence photometer (D1) and the device (D2) for the amperometric or photometric determination of the blood glucose level together define at least a measurement unit (70,71,72) and a calibration unit (80,81,82).

11. Apparatus according to claim 10, further comprising a communication and contacts unit (90), the calibration unit (80) being designed to provide a blood glucose measurement value representative of the blood glucose level, the communication and contacts unit (90) being designed to communicate the blood glucose measurement value to the measurement unit (70), and the measurement unit (70) being designed to self-calibrate using the communicated blood glucose measurement value.

12. Apparatus according to claim 10, wherein the calibration unit (81,82) is designed to provide a measurement value that is forwarded to the measurement unit (71,72), the measurement unit (71,72) being designed to convert the communicated measurement value into a blood glucose measurement value representative of the blood glucose level, and the measurement unit (71,72) being designed to self-calibrate using the blood glucose measurement value.

13. Apparatus according to claim 12, wherein the calibration unit (82) and the measurement unit (72) have a common digital control element (722), the digital control element (722) being provided in the measurement unit (72), the calibration unit (82) comprising only an analogue measurement circuit (821) controlled by the common digital control element (722).

## Patentansprüche

1. Kombinierte Vorrichtung (D1, D2; D3) zum Messen eines Blutglukoselevels in einer Augenflüssigkeit eines Benutzers, umfassend
- ein handgehaltenes Fluoreszenz-Photometer (D1) zum Bestimmen des Glukoselevels der Augenflüssigkeit des Benutzers
- eine Vorrichtung (D2) für die amperometrische oder photometrische Bestimmung des Blutglukoselevels des Benutzers zum Zweck einer Kalibration,
wobei das handgehaltene Fluoreszenz-Photometer (D1) umfasst:
(a) erste Bestrahlungsmittel (7, 8, 10) zum Bereitstellen eines Pilotstrahls (1) zur Bestrahlung des Auges (3) des Benutzers, um eine Pupillenfluoreszenz anzuregen, das heißt eine erste Fluoreszenz, umfassend ein erstes Wellenlängenband, wobei die Pupillenfluoreszenz sich entlang eines ersten optischen Weges ausbreitet;
(b) erste Detektormittel (8, 9, 12, 13), angeordnet in dem ersten optischen Weg, zum Messen der Intensität der Pupillenfluoreszenz innerhalb des ersten Wellenlängenbands;
(c) zweite Bestrahlungsmittel (17, 18, 21) zum Bereitstellen eines Messstrahls (5) zum Bestrahlen des Auges (3) des Benutzers, um einen Augensensor (4) anzuregen, der in Kontakt steht mit der Augenflüssigkeit des Benutzers und derbei Bestrahlung mit dem Messstrahl - eine zweite Fluoreszenz emittiert, die ein zweites Wellenlängenband umfasst, wobei die zweite Fluoreszenz sich entlang eines zweiten optischen Weges ausbreitet;
(d) zweite Detektormittel (18, 19, 20, 22, 23, 24, 25), die in dem zweiten optischen Weg angeordnet sind, zur Messung der Intensität der zweiten Fluoreszenz innerhalb des zweiten Wellenlängenbands;
wobei die ersten und zweiten Bestrahlungsmittel derart angeordnet sind, dass der Messstrahl (5) das Auge des Benutzers in einem vorgegebenen Winkel (α) bestrahlt und in einer vorgegebenen Distanz von dem Pilotstrahl (1), wobei der Winkel (α) zwischen 0° und 90° liegt.

2. Vorrichtung nach Anspruch 1, wobei der vorgegebene Winkel (α) und die Distanz derart gewählt sind, dass, sobald das Fluoreszenz-Photometer (D1) in einer geeigneten Distanz und Orientierung vor dem Auge des Benutzers angeordnet ist, der Messstrahl (5) auf die Iris (6) auftrifft, während der Pilotstrahl auf die Pupille (2) auftrifft.

3. Vorrichtung nach einem der vorhergehenden Ansprüche, wobei die ersten Detektormittel einen ersten Detektor (13) zur Detektion der Pupillenfluoreszenz oder ersten Fluoreszenz und mindestens ein wellenlängenselektives Element (8, 9, 12) umfassen, welches in dem ersten optischen Weg angeordnet ist, um zu verhindern, dass Licht, das eine Wellenlänge außerhalb des ersten Wellenlängenbands aufweist, auf dem ersten Detektor (13) auftrifft.

4. Vorrichtung nach einem der vorhergehenden Ansprüche, wobei die zweiten Detektormittel mindestens einen zweiten Detektor (24) zum Detektieren der zweiten Fluoreszenz, die von dem Augensensor (4) emittiert wird, aufweisen, und mindestens ein wellenlängenselektives Element (18, 19, 22), um zu verhindern, dass Licht, welches eine Wellenlänge außerhalb des zweiten Wellenlängenbands aufweist, auf den zweiten Detektor (24) auftrifft.

5. Vorrichtung nach Anspruch 4, wobei die zweiten Detektormittel weiterhin einen dritten Detektor (25) aufweisen, zum Detektieren einer dritten Fluoreszenz, die von dem Augensensor (4) emittiert wird und mindestens ein wellenlängenselektives Element (18, 19, 20, 23), um zu verhindern, dass Licht, welches eine Wellenlänge außerhalb des dritten Wellenlängenbands aufweist, auf den dritten Detektor (25) auftrifft.

6. Vorrichtung nach einem der vorhergehenden Ansprüche, wobei das handgehaltene Fluoreszenz-Photometer (D1) und die Vorrichtung (D2) für die amperometrische oder photometrische Bestimmung des Blutglukoselevels zusammen eine einzelne integrale Vorrichtung (D3) bilden.

7. Vorrichtung nach Anspruch 6, wobei die einzelne integrale Vorrichtung (D3) die Gestalt einer Scheibe aufweist, die einen Durchmesser zwischen 3 und 20 cm aufweist, vorzugsweise zwischen 5 und 15 cm und am meisten bevorzugt zwischen 7 und 10 cm, wobei die Scheibe eine Dicke zwischen 1 und 7 cm aufweist, vorzugsweise zwischen 2 und 4 cm.

8. Vorrichtung nach einem der Ansprüche 1 bis 5, wobei das handgehaltene Fluoreszenz-Photometer (D1) und die Vorrichtung (D2) für die amperometrische oder photometrische Bestimmung des Blutglukoselevels getrennte Vorrichtungen sind, welche verbunden werden können, um in Kombination miteinander zu stehen.

9. Vorrichtung nach Anspruch 8, wobei die Vorrichtung (D2) für die amperometrische oder photometrische Bestimmung des Blutglukoselevels ausgestaltet ist als Docking-Station, an welche das handgehaltene Fluoreszenz-Photometer (D1) angedockt werden kann.

10. Vorrichtung nach einem der vorhergehenden Ansprüche, wobei das handgehaltene Fluoreszenz-Photometer (D1) und die Vorrichtung (D2) für die amperometrische oder photometrische Bestimmung des Blutglukoselevels zusammen zumindest eine Messeinheit (70, 71, 72) und eine Kalibrationseinheit (80, 81, 82) definieren.

11. Vorrichtung nach Anspruch 10, weiterhin umfassend eine Kommunikations- und Kontakteinheit (90), wobei die Kalibrationseinheit (80) eingerichtet ist, um einen Blutglukose-Messwert zu liefern, welcher repräsentativ ist für den Blutglukoselevel,
wobei die Kommunikations- und Kontakteinheit (90) ausgestaltet ist, um den Blutglukose-Messwert an die Messeinheit (70) zu liefern, und wobei die Messeinheit (70) eingerichtet ist, um sich unter Verwendung des kommunizierten Blutglukose-Messwertes selbst zu kalibrieren.

12. Vorrichtung nach Anspruch 10, wobei die Kalibrationseinheit (81, 82) eingerichtet ist, um einen Messwert zu liefern, welcher an die Messeinheit (71, 72) weitergeleitet wird, wobei die Messeinheit (71, 72) ausgestaltet ist, um den kommunizierten Messwert in einen Blutglukose-Messwert umzuwandeln, welcher repräsentativ ist für den Blutglukoselevel, und wobei die Messeinheit (71, 72) eingerichtet ist, um unter Verwendung des Blutglukose-Messwertes sich selbst zu kalibrieren.

13. Vorrichtung nach Anspruch 12, wobei die Kalibrationseinheit (82) und die Messeinheit (72) ein gemeinsames digitales Steuerelement (722) aufweisen, wobei das digitale Steuerelement (722) in der Messeinheit (72) vorgesehen ist, wobei die Kalibrationseinheit (82) nur einen analogen Messschaltkreis (821) aufweist, der durch das gemeinsame digitale Steuerelement (722) gesteuert wird.

## Revendications

1. Appareil combiné (D1, D2 ; D3) pour mesurer le niveau de glycémie à partir d'un liquide oculaire d'un utilisateur, comprenant
- un photomètre de fluorescence portatif (D1) pour déterminer le niveau de glucose du liquide oculaire de l'utilisateur,
- un dispositif (D2) pour la détermination ampérométrique ou photométrique du niveau de glycémie de l'utilisateur à des fins d'étalonnage,
dans lequel le photomètre de fluorescence portatif (D1) comprend :
(a) un premier moyen d'irradiation (7, 8, 10) pour fournir un faisceau pilote (1) pour irradiation de l'oeil (3) de l'utilisateur afin d'exciter une fluorescence de pupille, c'est-à-dire une première fluorescence, comprenant une première bande de longueurs d'onde, ladite fluorescence de pupille se déplaçant le long d'un premier chemin optique ;
(b) un premier moyen de détection (8, 9, 12, 13) disposé sur le premier chemin optique pour mesurer l'intensité de la fluorescence de pupille à l'intérieur de la première bande de longueurs d'onde ;
(c) un deuxième moyen d'irradiation (17, 18, 21) pour fournir un faisceau de mesure (5) pour irradiation de l'oeil (3) de l'utilisateur afin d'exciter un capteur oculaire (4) qui est en contact avec le liquide oculaire de l'utilisateur et qui - lors de l'irradiation avec ledit faisceau de mesure - émet une deuxième fluorescence comprenant une deuxième bande de longueurs d'onde, ladite deuxième fluorescence se déplaçant le long d'un deuxième chemin optique ;
(d) un deuxième moyen de détection (18, 19, 20, 22, 23, 24, 25) disposé sur le deuxième chemin optique pour mesurer l'intensité de la deuxième fluorescence à l'intérieur de la deuxième bande de longueurs d'onde ;
dans lequel le premier et le deuxième moyen d'irradiation sont disposés de telle sorte que le faisceau de mesure (5) irradie l'oeil de l'utilisateur sous un angle prédéterminé (α) et à une distance prédéterminée du faisceau pilote (1), ledit angle (α) se situant entre 0° et 90°.

2. Appareil selon la revendication 1, dans lequel l'angle (α) et la distance prédéterminés sont choisis de telle sorte qu'une fois le photomètre de fluorescence (D1) disposé à une bonne distance et dans une orientation correcte devant l'oeil de l'utilisateur, le faisceau de mesure (5) frappe l'iris (6) tandis que le faisceau pilote frappe la pupille (2).

3. Appareil selon l'une quelconque des revendications précédentes, dans lequel le premier moyen de détection comprend un premier détecteur (13) pour détecter la fluorescence de pupille ou première fluorescence et au moins un élément sélectif en longueur d'onde (8, 9, 12) disposé sur le premier chemin optique pour empêcher la lumière ayant une longueur d'onde à l'extérieur de la première bande de longueurs d'onde de frapper le premier détecteur (13).

4. Appareil selon l'une quelconque des revendications précédentes, dans lequel le deuxième moyen de détection comprend au moins un deuxième détecteur (24) pour détecter la deuxième fluorescence émise par le capteur oculaire (4) et au moins un élément sélectif en longueur d'onde (18, 19, 22) pour empêcher la lumière ayant une longueur d'onde à l'extérieur de la deuxième bande de longueurs d'onde de frapper le deuxième détecteur (24).

5. Appareil selon la revendication 4, dans lequel le deuxième moyen de détection comprend en outre un troisième détecteur (25) pour détecter une troisième fluorescence émise par le capteur oculaire (4) et au moins un élément sélectif en longueur d'onde (18, 19, 20, 23) pour empêcher la lumière ayant une longueur d'onde à l'extérieur de la troisième bande de longueurs d'onde de frapper le troisième détecteur (25).

6. Appareil selon l'une quelconque des revendications précédentes, dans lequel le photomètre de fluorescence portatif (D1) et le dispositif (D2) pour la détermination ampérométrique ou photométrique du niveau de glycémie forment conjointement un dispositif intégral unique (D3).

7. Appareil selon la revendication 6, dans lequel le dispositif intégral unique (D3) a la forme d'un disque ayant un diamètre compris entre 3 et 20 cm, de préférence entre 5 et 15 cm, et idéalement entre 7 et 10 cm, le disque ayant une épaisseur comprise entre 1 et 7 cm, de préférence entre 2 et 4 cm.

8. Appareil selon l'une quelconque des revendications 1 à 5, dans lequel le photomètre de fluorescence portatif (D1) et le dispositif (D2) pour la détermination ampérométrique ou photométrique du niveau de glycémie sont des dispositifs distincts, qui peuvent être reliés afin d'être en communication l'un avec l'autre.

9. Appareil selon la revendication 8, dans lequel le dispositif (D2) pour la détermination ampérométrique ou photométrique du niveau de glycémie est conçu comme une station d'accueil à laquelle le photomètre de fluorescence portatif (D1) peut être arrimé.

10. Appareil selon l'une quelconque des revendications précédentes, dans lequel le photomètre de fluorescence portatif (D1) et le dispositif (D2) pour la détermination ampérométrique ou photométrique du niveau de glycémie définissent conjointement au moins une unité de mesure (70, 71, 72) et une unité d'étalonnage (80, 81, 82).

11. Appareil selon la revendication 10, comprenant en outre une unité de communication et de contacts (90), l'unité d'étalonnage (80) étant conçue pour fournir une valeur de mesure de la glycémie représentative du niveau de glycémie, l'unité de communication et de contacts (90) étant conçue pour communiquer la valeur de mesure de la glycémie à l'unité de mesure (70), et l'unité de mesure (70) étant conçue pour s'auto-étalonner en utilisant la valeur de mesure de la glycémie communiquée.

12. Appareil selon la revendication 10, dans lequel l'unité d'étalonnage (81, 82) est conçue pour fournir une valeur de mesure qui est transférée à l'unité de mesure (71, 72), l'unité de mesure (71, 72) étant conçue pour transformer la valeur de mesure communiquée en une valeur de mesure de la glycémie représentative du niveau de glycémie, et l'unité de mesure (71, 72) étant conçue pour s'auto-étalonner en utilisant la valeur de mesure de la glycémie.

13. Appareil selon la revendication 12, dans lequel l'unité d'étalonnage (82) et l'unité de mesure (72) comportent un élément de commande numérique (722) commun, l'élément de commande numérique (722) étant pourvu dans l'unité de mesure (72), l'unité d'étalonnage (82) comprenant uniquement un circuit de mesure analogique (821) contrôlé par l'élément de commande numérique (722) commun.
